(19) Europäisches Patentamt
European Patent Office
Office européen des brevets

(11) Veröffentlichungsnummer: **0 019 133**
**B1**

(12) # EUROPÄISCHE PATENTSCHRIFT

(45) Veröffentlichungstag der Patentschrift: **20.07.83**

(51) Int. Cl.³: **A 01 N 43/64,** C 07 D 249/08

(21) Anmeldenummer: **80102292.2**

(22) Anmeldetag: **28.04.80**

(54) **Fungizide Mittel, Verfahren zu ihrer Herstellung sowie ihre Verwendung als Fungizide.**

(30) Priorität: **08.05.79 DE 2918467**

(43) Veröffentlichungstag der Anmeldung: **26.11.80 Patentblatt 80/24**

(45) Bekanntmachung des Hinweises auf die Patenterteilung: **20.07.83 Patentblatt 83/29**

(84) Benannte Vertragsstaaten: **AT BE CH DE FR GB IT LI NL**

(56) Entgegenhaltungen:
**EP-A-0 004 917**
**DE-A-2 723 942**
**FR-A-2 303 475**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal-1 (DE)**
Erfinder: **Knops, Hans-Joachim, Dr., Claudiusweg 3, D-5600 Wuppertal-1 (DE)**
Erfinder: **Büchel, Karl-Heinz, Dr., Bergerheide 62, D-5600 Wuppertal-1 (DE)**
Erfinder: **Brandes, Wilhelm, Dr., Eichendorffstrasse 3, D-5623 Leichlingen (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Fungizide Mittel, Verfahren zu ihrer Herstellung sowie ihre Verwendung als Fungizide

Die vorliegende Erfindung betrifft die Verwendung der teilweise bekannten 1-(2,4-Dichlorphenyl)-1-(2,6-dihalogenbenzyloximino)-2-(1,2,4-triazol-1-yl)-ethane als selektive Fungizide gegen Botrytispilze.

Es ist bereits bekannt geworden, dass 1-(2,4-Dichlorphenyl)-1-(halogenbenzyloximino)-2-(1,2,4-triazol-1-yl)-ethane eine sehr gute fungizide Wirksamkeit besitzen (vergleiche DE-OS 2 723 942). Über die vom wirtschaftlichen Standpunkt aus hoch interessante Wirksamkeit gegenüber Botrytispilzen ist jedoch nichts bekannt.

Es wurde gefunden, dass speziell die 1-(2,4-Dichlorphenyl)-1-(2,6-dihalogenbenzyloximino)-2-(1,2,4-triazol-1-yl)-ethane der Formel

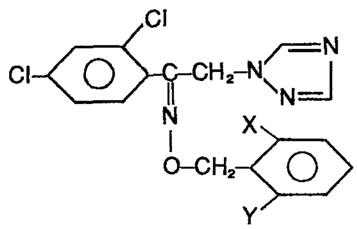

(I)

in welcher

X und Y gleich oder verschieden sind und für Halogen stehen,

und deren physiologisch verträgliche Säureadditions-Salze eine besonders gute selektive fungizide Wirksamkeit gegen Botrytispilze aufweisen.

Die Verbindungen der Formel (I) können in der syn- oder anti-Form vorliegen; vorwiegend fallen sie als Gemische beider Formen an.

Überraschenderweise zeigen die 1-(2,4-Dichlorphenyl)-1-(2,6-dihalogenbenzyloximino)-2-(1,2,4-triazol-1-yl)-ethane der Formel (I) eine erheblich höhere selektive fungizide Wirkung als die aus dem Stand der Technik bekannten vergleichbaren 1-(2,4-Dichlorphenyl)-1-(halogenbenzyloximino)-2-(1,2,4-triazol-1-yl)-ethane, wie beispielsweise die entsprechenden 2,4-Dichlorbenzyloximino-, 4-Chlorbenzyloximino- und 2-Chlorbenzyloximino-Derivate, welche chemisch die naheliegendsten Verbindungen sind.

Die neue Verwendung der Wirkstoffe stellt somit eine Bereicherung der Technik dar.

Die erfindungsgemäss verwendbaren Verbindungen sind durch die Formel (I) definiert. In dieser Formel stehen X und Y vorzugsweise für Chlor und/oder Fluor. Die Verbindungen sind teilweise bekannt (vergleiche DE-OS 2 723 942). Noch nicht bekannt sind diejenigen Verbindungen der Formel (I), in denen X und Y nicht gleichzeitig für Chlor stehen. Interessante neue Verbindungen sind somit diejenigen Stoffe der Formel (I), in denen X Chlor oder Fluor bedeutet, während Y auf Fluor festgelegt ist.

Die Verbindungen der Formel (I) können in bekannter Art und Weise erhalten werden, indem man in einer ersten Stufe ω-Halogen-2,4-dichloracetophenone mit 1,2,4-Triazol in Gegenwart eines inerten organischen Lösungsmittels und in Gegenwart eines Säurebinders bei Temperaturen zwischen 20 und 120 °C umsetzt; die entstehenden ω-(1,2,4-Triazol-1-yl)-2,4-dichloracetophenone in einer zweiten Stufe mit Hydroxylamin in Gegenwart eines Lösungsmittels, vorzugsweise Alkohole, bei 50 bis 100 °C umsetzt, wobei das Hydroxylamin vorzugsweise als Hydrochlorid in Gegenwart eines Säurebindemittels eingesetzt wird, und die entstehenden ω-(1,2,4-Triazol-1-yl)-2,4-dichloracetophenon-oxime in einer dritten Stufe in Gegenwart eines inerten organischen Lösungsmittels und gegebenenfalls in Gegenwart einer starken Base bei Temperaturen zwischen 20 und 100 °C mit 2,6-Dihalogenbenzylhalogeniden umsetzt (vergleiche hierzu auch die Angaben in DE-OS 2 657 578 und DE-OS 2 723 942).

Die erfindungsgemäss zu verwendenden Stoffe der Formel (I) können auch nach einem eigenen, neuen Verfahren erhalten werden, das nicht zum Stand der Technik gehört (vergleiche die deutsche Patentanmeldung P 2 907 972.9 vom 01.03.1979 [Le A 19 473]), indem man ω-Halogen-dichloracetophenone der Formel

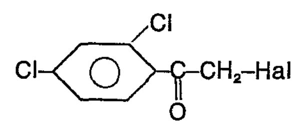

(II)

in welcher

Hal für Halogen, vorzugsweise Chlor oder Brom, steht,

mit Hydroxylaminen der Formel

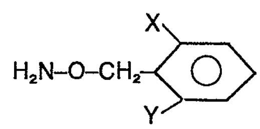

(III)

in welcher

X und Y die oben angegebene Bedeutung haben,

in Gegenwart eines Verdünnungsmittels, wie vorzugsweise Alkohole bzw. wässrige Alkohole, bei Temperaturen zwischen 50 und 100 °C umsetzt, wobei die Hydroxylamine der Formel (III) vorzugsweise in Form ihrer Hydrochloride in Gegenwart eines Säurebindemittels eingesetzt werden; und die dabei entstehenden ω-Halogen-2,4-dichloracetophenon-oximether der Formel

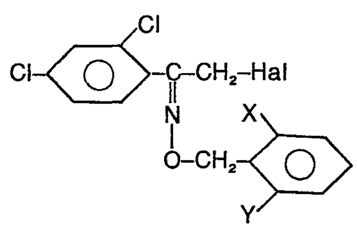

(IV)

in welcher

X, Y und Hal die oben angegebene Bedeutung haben,

mit 1,2,4-Triazol in Gegenwart eines organischen Lösungsmittels und in Gegenwart eines Säurebindemittels, wie beispielsweise Kaliumcarbonat, bei Temperaturen zwischen 60 bis 120 °C umsetzt. In einer besonderen Ausführungsform kann dieses Verfahren auch ohne Isolierung der Zwischenprodukte der Formel (IV) und ohne Lösungsmittelwechsel als sogenanntes Eintopfverfahren durchgeführt werden (vergleiche auch die Herstellungsbeispiele).

Zur Herstellung von Säureadditionssalzen der Verbindungen der Formel (I) kommen alle physiologisch verträglichen Säuren in Frage. Hierzu gehören vorzugsweise die Halogenwassserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäure und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Eine besonders gute Wirksamkeit entfalten die erfindungsgemässen Wirkstoffe gegen Botrytispilze, z.B. gegen Botrytis cinerea, den Erreger des Grauschimmels an Gartenbohnen, Salat, Erdbeeren und Reben.

Die Wirkstoffe können in die üblichen Formulierungen übergeführt werden, wie Lösungen, Emulsionen, Suspensionen, Pulver, Schäume, Pasten, Granulate, Aerosole, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromate, wie Xylol, Toluol oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können in den Formulierungen oder in den verschiedenen

Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gewichtsprozent, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gewichtsprozent, vorzugsweise von 0,0001 bis 0,02%, am Wirkungsort erforderlich.

Beispiel A

Botrytis-Test (Salat)/protektiv

| | |
|---|---|
| Lösungsmittel: | 4,7 Gewichtsteile Aceton |
| Dispergiermittel: | 0,3 Gewichtsteile Alkylarylpolygykolether |
| Wasser: | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man Pflanzen von Kopfsalat im 8-Blattstadium bis zur Tropfnässe. Nach 24 Stunden werden auf jedes Blatt 2 kleine mit Botrytis cinerea bewachsene Agarstükke aufgelegt. Die inokulierten Pflanzen werden in einer abgedunkelten, feuchten Kammer bei 20 °C aufgestellt. 3 Tage nach der Inokulation wird die Grösse der Befallsflecken auf den Blättern bonitiert.

Die erhaltenen Boniturwerte werden in Prozent Befall umgerechnet. 0% bedeutet keinen Befall, 100% bedeutet, dass der Befallsfleck vollständig ausgebildet ist.

Wirkstoffe, Wirkstoffkonzentrationen und Ergebnisse gehen aus der nachfolgenden Tabelle hervor:

Tabelle A

Botrytis-Test (Salat) / protektiv

| Wirkstoff | Befall in % bei einer Wirkstoffkonzentration von 0,025% |
|---|---|
| 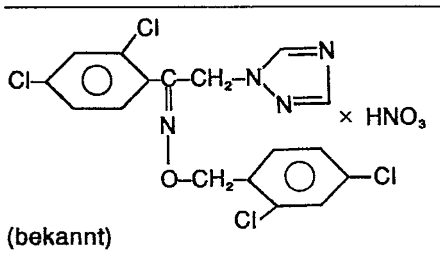 (bekannt) | 100 |
| 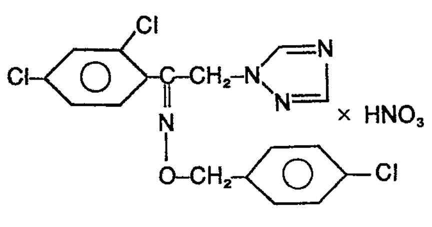 (bekannt) | 100 |
| 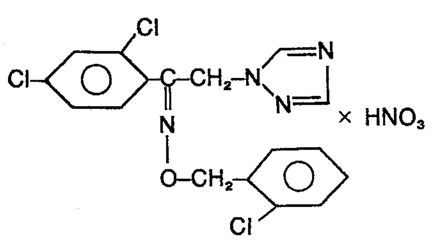 (bekannt) | 96 |

Tabelle A Fortsetzung
Botrytis-Test (Salat) / protektiv

| Wirkstoff | Befall in % bei einer Wirkstoffkonzentration von 0,025% |
|---|---|
| 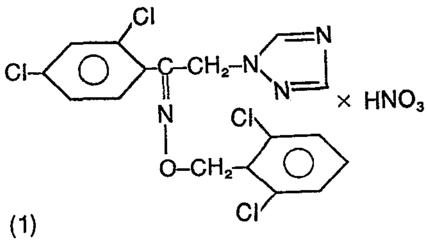 (1) | 9 |
| 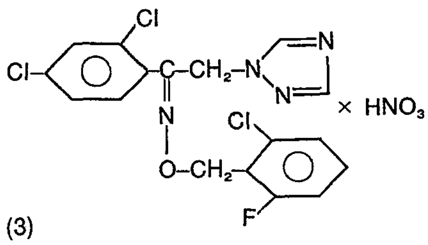 (3) | 7 |

## Herstellungsbeispiele

### Beispiel 1

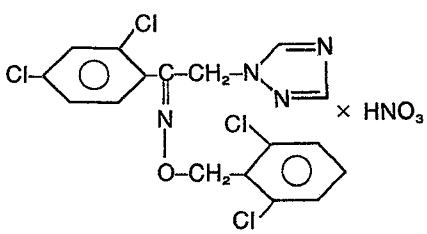

27,1 g (0,1 Mol) ω-(1,2,4-Triazol-1-yl)-2,4-dichlor-acetophenon-oxim werden in 300 ml Toluol suspendiert, 300 ml 45%ige Natronlauge zugegeben und 3 ml Benzyl-dimethylammoniumchlorid zugesetzt. Man tropft unter Rühren 43 g (etwa 0,22 Mol) 2,6-Dichlorbenzylchlorid zu und rührt 10 Stunden bei 40 °C. Die Phasen werden getrennt, die organische Phase wird zweimal mit je 300 ml gesättigter Natriumchloridlösung gewaschen, über Natriumsulfat getrocknet und das Lösungsmittel im Wasserstrahlvakuum abdestilliert. Der Rückstand wird in 100 ml Chloroform aufgenommen, unter Eiskühlung werden 3 ml konzentrierter Salpetersäure zugetropft. Man lässt auskristallisieren, saugt den Niederschlag ab und wäscht zweimal mit 50 ml Diethylether nach. Man erhält 23 g (42% der Theorie) ω-(1,2,4-Triazol-1-yl)-2,4-dichloracetophenon-oxim-2,6-dichlorbenzyl-ether-nitrat vom Schmelzpunkt 160 °C (Zers.).

### Beispiel 2

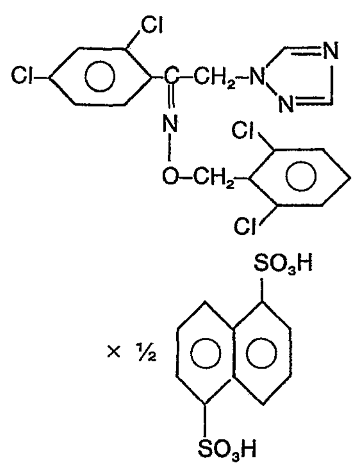

8,9 g (0,04 Mol) ω-Chlor-2,4-dichloracetophenon werden mit 8,6 g (0,04 Mol) 0-2,6-Dichlorbenzyl-hydroxylamin-hydrochlorid und 3,3 g (0,04 Mol) Natriumacetat in 100 ml Ethanol 15 Stunden unter Rückfluss erhitzt. Zu diesem Reaktionsgemisch werden 2,8 g (0,04 Mol) 1,2,4-Triazol und 5,6 g (0,04 Mol) Kaliumcarbonat gegeben und weitere 20 Stunden unter Rückfluss erhitzt. Nach dem Erkalten wird filtriert und das Filtrat eingeengt. Der Rückstand wird in 250 ml Methylenchlorid aufgenommen, fünfmal mit je 200 ml Wasser gewaschen, über Natriumsulfat getrocknet und eingeengt. Man erhält 15,4 g (89,5% der Theorie) ω-(1,2,4-Triazol-1-yl)-2,4-dichloracetophenon-oxim-0-2,6-dichlorbenzyl-ether als Öl. Dieses wird zur

Salzbildung in Aceton gelöst. Man filtriert eine Lösung von 5,4 g 1,5-Naphthalindisulfonsäure in 25 ml Aceton zu, verrührt 10 Minuten bei Raumtemperatur und saugt den Niederschlag ab. Man erhält 14,8 g (72% der Theorie – bezogen auf eingesetzte Base) ω-(1,2,4-Triazol-1-yl)-2,4-dichloracetophenon-oxim-2,6-dichlorbenzyl-ether-1,5-naphthalindisulfonat vom Schmelzpunkt 245–252 °C (Zers.).

In entsprechender Weise können die folgenden Verbindungen der allgemeinen Formel

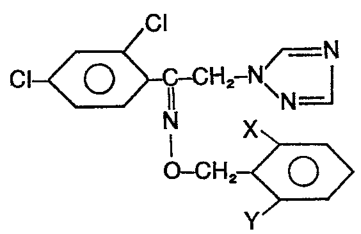

(I)

erhalten werden:

| Bsp. Nr. | X | Y | Schmelzpunkt (°C) |
|---|---|---|---|
| 3 | Cl | F | 135 (Zers.) (x$HNO_3$) |
| 4 | F | F | |

**Patentansprüche**

1. Verfahren zur Bekämpfung von Botrytispilzen, dadurch gekennzeichnet, dass man 1-(2,4-Dichlorphenyl)-1-(2,6-dihalogenbenzyloximino)-2-(1,2,4-triazol-1-yl)-ethane der Formel

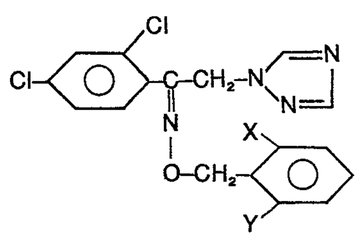

(I)

in welcher

X und Y gleich oder verschieden sind und für Halogen stehen,

oder deren physiologisch verträgliche Säureadditionssalze auf Botrytispilze oder ihren Lebensraum wirken lässt.

2. Verwendung von 1-(2,4-Dichlorphenyl)-1-(2,6-dihalogenbenzyloximino)-2-(1,2,4-triazol-1-yl)-ethane der Formel (I) zur Bekämpfung von Botrytispilzen.

3. 1-(2,4-Dichlorphenyl)-1-(2,6-dihalogenbenzyloximino)-2-(1,2,4-triazol-1-yl)-ethane der Formel (I), in welcher

X für Chlor oder Fluor und

Y für Fluor stehen.

**Claims**

1. Process for combating Botrytis fungi, characterised in that 1-(2,4-dichlorophenyl)-1-(2,6-dihalogenobenzyloximino)-2-(1,2,4-triazol-1-yl)-ethanes of the formula

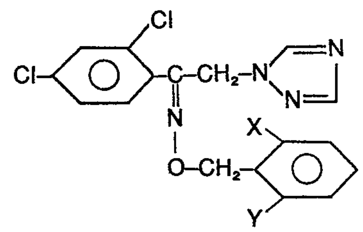

(I)

in which

X and Y are identical or different and represent halogen,

or physiologically acceptable acid addition salts thereof are allowed to act on Botrytis fungi or their environment.

2. Use of 1-(2,4-dichlorophenyl)-1-(2,6-dihalogenobenzyloximino)-2-(1,2,4-triazol-1-yl)-ethanes of the formula (I) for combating Botrytis fungi.

3. 1-(2,4-Dichlorophenyl)-1-(2,6-dihalogenobenzyloximino)-2-(1,2,4-triazol-1-yl)-ethanes of the formula (I), in which

X represents chlorine or fluorine and

Y represents fluorine.

**Revendications**

1. Procédé en vue de combattre les champignons de l'espèce Botrytis, caractérisé en ce qu'on fait agir des 1-(2,4-dichlorophényl)-1-(2,6-dihalogénobenzyloximino)-2-(1,2,4-triazol-1-yl)-éthanes de formule

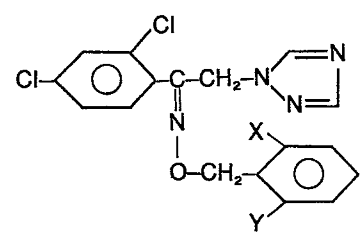

(I)

dans laquelle

X et Y sont identiques ou différents et représentent chacun un atom d'halogène,

ou leurs sels d'addition d'acide physiologiquement compatibles sur des champignons Botrytis ou leurs biotopes.

2. Utilisation de 1-(2,4-dichlorophényl)-1-(2,6-dihalogénobenzyloximino)-2-(1,2,4-triazol-1-yl)-éthanes de formule (I) pour combattre des champignons de l'espèce Botrytis.

3. 1-(2,4-dichlorophényl)-1-(2,6-dihalogénobenzyloximino)-2-(1,2,4-triazol-1-yl)-éthanes de formule (I) dans laquelle

X représente un atome de chlore ou un atome de fluor et

Y représente un atome de fluor.